# EUROPEAN PATENT APPLICATION

(11) **EP 4 592 949 A1**
(43) Date of publication of application: **30.07.2025**
(21) Application number: 25152978.0
(22) Date of filing: 21.01.2025
(51) Int. Cl.: G06T 7/00, G16H 20/40, G16H 30/40

(54) **AUTOMATED DETERMINATION OF BONE MINERAL DENSITY FROM A MEDICAL IMAGE**

(30) Priority: 25.01.2024 US 202463624932 P; 18.10.2024 US 202463709057 P; 26.11.2024 US 202463725156 P
(71) Applicant: MAKO Surgical Corp., Weston, FL 33331 (US)
(72) Inventor: BRETT, Alan Donald, Austin, TX, 78746 (US); MAGUIRE, Niall Collum, Cadisheas, Manchester, M44 5YW (GB)
(74) Representative: Röthinger, Rainer

(57) **Abstract**

A computer-implemented method for evaluating a medical image exhibiting air, fat, a bone, and a motion rod. The computer-implemented method includes automatically performing the following: identifying, from the medical image, intensities of the bone, the motion rod and at least one of: air or fat; obtaining a bone mineral density equivalent value of the motion rod and the at least one of the air or the fat; determining a bone mineral density calibration factor based on a relationship between the identified intensities and a bone mineral density equivalent values of the motion rod and the at least one of the air or the fat; and determining the bone mineral density of the bone within the medical image based on the bone mineral density calibration factor.

## Description

### CROSS-REFERENCE TO RELATED APPLICATIONS

The present application claims priority to and all the benefits of U.S. Provisional Patent Application No. 63/624,932, filed January 25, 2024, U.S. Provisional Patent Application No. 63/709,057, filed on October 18, 2024, and U.S. Provisional Patent Application No. 63/725,156, filed on November 26, 2024, wherein the contents of each of the above referenced applications are hereby expressly incorporated herein by reference in their entirety.

### BACKGROUND

Understanding the bone mineral density (BMD) from medical images is crucial for various medical treatments, including bone health assessments, risk predictions, treatment monitoring, and preoperative planning for surgeries such as knee or hip replacements. When planning knee joint replacement surgeries, several factors influence the choice of implantation techniques, including the type of screws, saw, and drill speeds. These decisions are based on numerous factors such as the patient's age, activity level, surgeon's experience, history of infections, bone quality, and implant type. The BMD value is a significant factor that supports the surgeon's decision-making process.

Patients with reduced BMD are at a higher risk of poor outcomes after joint replacement surgery. For instance, cementless implants, which rely on press-fit fixation in the surrounding bone for primary stability, may not perform well in patients with low BMD. Traditionally, surgeons have used a "thumb test" to manually judge bone strength by pressing with the thumb. However, this method is subjective and lacks precision.

BMD measurement for diagnosing low bone mass and osteoporosis is typically performed using dual-energy X-ray absorptiometry (DEXA) scanning. However, BMD values cannot be directly derived from computed tomography (CT) scans without additional calibration. Internal reference standards used in calibration can be affected by the hydration state and the dynamic BMD range spanned by the tissues.

CT scans generate medical images, usually in grayscale, representing data in Hounsfield Units (HU). Hounsfield Units measure the attenuation of X-rays as they pass through different tissues, with water assigned a value of 0 HU and air around -1000 HU. To calibrate BMD values from CT scans, a BMD calibration phantom, typically a plastic block with embedded rods of different bone density equivalents, is used. This phantom is placed under the area of interest during the scan. The calibration process converts the Hounsfield Units measured in the CT scanner to BMD values. However, in clinical practice, these phantoms are often not readily available, can be lost, and are uncomfortable for patients, hindering the BMD calculation process.

Therefore, there is a need for an accessible, accurate, and reliable calibration method that allows for BMD calculation from CT scans without the need for a physical phantom.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a computer-implemented method is provided for evaluating a medical image exhibiting air, fat, a bone, and a motion rod. The computer-implemented method includes automatically performing the following: identifying, from the medical image, intensities of the bone, the motion rod and at least one of: air or fat; obtaining a bone mineral density equivalent value of the motion rod and the at least one of the air or the fat; determining a bone mineral density calibration factor BCF based on a relationship between the identified intensities and a bone mineral density equivalent values of the motion rod and the at least one of the air or the fat; and determining the bone mineral density of the bone within the medical image based on the bone mineral density calibration factor.

According to a second aspect, a non-transitory computer readable medium is provided comprising instructions configured to automatically determine bone mineral density (BMD) from a medical image exhibiting air, fat and a motion rod, wherein the instructions, when executed by the one or more processors, are configured automatically identify, from the medical image, intensities of the bone, the motion rod and at least one of: air or fat; automatically obtain a bone mineral density equivalent value of the motion rod and the at least one of the air or the fat; automatically determine a BMD calibration factor based on a relationship between the identified intensities and a BMD equivalent values of the motion rod and the at least one of the air or the fat; and automatically determine the BMD of the bone within the medical image based on the BMD calibration factor.

According to a third aspect, a computer-implemented method or non-transitory computer readable medium is provided to automatically determine a bone mineral density (BMD) value of a bone in a medical image by being configured to determine a relationship between image parameters of the bone, air, fat, and a motion rod in the medical image.

According to a fourth aspect, a computer-implemented method for evaluating a medical image exhibiting air, fat, bone, and a motion rod is presented. The computer-implemented method includes the following: identifying, from the medical image, an image voxel containing air; identifying, from the medical image, a motion rod surface; identifying, from the medical image, a bone surface; determining a normal ray projected from the bone surface; identifying, from the normal ray, the fat; determining the intensities of the bone surface, the motion rod surface and at least one of: the air voxel or the fat; obtaining the bone mineral density equivalent value of the motion rod and the at least one of the air or the fat; determining a BMD calibration factor based on a relationship between the identified intensities and a BMD equivalent values of the motion rod and the at least one of the air or the fat; and determining the BMD of the bone within the medical image based on the BMD calibration factor.

Also provided are a non-transitory computer-readable medium comprising instructions, which when executed by one or more processors, operate the computer-implemented method of the fourth aspect.

Any of the above aspects may be combined, in whole or in part.

Any of the above aspects may be combined with any of the following implementations. Any of the following implementations may be utilized in part, or in whole, with any of the above aspects. The implementations include, but are not limited to:

The computer implemented method further including identifying, from the medical image, intensities of the bone, the motion rod, the air, and the fat; obtaining a bone mineral density equivalent value of the motion rod, the air, and the fat; determining a BMD calibration factor based on a relationship between the identified intensities and the BMD equivalent values of the motion rod, the air, and the fat; and determining the BMD of the bone within the medical image based on the BMD calibration factor.

The method may further include generating from the medical image a 3D model of the bone comprising a mesh surface. The method may further include identifying, from the medical image a skin boundary; generating vectors normal to the mesh surface of the bone and towards the skin boundary; identifying an intensity distribution of the vectors; identifying a fat peak based on the intensity distribution; and identifying the intensity of the fat based on the fat peak. The BMD equivalent value of the motion rod, the air, and the fat may be predetermined. The relationship between the identified intensities and the BMD equivalent values may be a linear relationship.

The method may further include generating a three-dimensional bone model exhibiting the determined BMD of the bone. The method may include generating a new medical image based on the BMD calibration factor; or calibrating the medical image based om the BMD calibration factor. The method may further include comparing the intensity and the BMD equivalent value of the one of the air or the fat not used in determining the BMD calibration factor to the relationship; and rejecting the BMD calibration factor as being unacceptable in response to a determination that the comparison exceeds a threshold. The medical image may be taken from a computed tomography scan. The motion rod may be a physical bar coupled to a patient's anatomy. Th motion rod may be comprised of aluminum. The method may further comprise determining the motion rod or the bone in the medical image using a statistical shape model or an active appearance model.

The method may further include controlling a surgical system such as a navigated robotic system that supports a tool that manipulates a bone. The navigated robotic system may manipulate the bone based on a determined bone mineral density of the bone.

### BRIEF DESCRIPTION OF THE DRAWINGS

Advantages of the present invention will be readily appreciated as the same becomes better understood by reference to the following detailed description when considered in connection with the accompanying drawings.
Figure 1 is a schematic diagram depicting an electronic data processing system having an image analysis system, according to the teaching of the present disclosure;
Figure 2 is a schematic diagram of the electronic data processing system of FIG. 1 depicting interactions among preoperative measurement systems, preoperative data, the image analysis system, outputs, and output systems, according to the teachings of the present disclosure;
Figure 3 illustrates prior art of a side view of a leg including a motion rod and the bone mineral density calibration phantom for the image analysis system, according to the teachings of the present disclosure;
Figure 4 illustrates prior art of a perspective view of the bone mineral density calibration phantom, according to the teachings of the present disclosure;
Figure 5 illustrates a medical image from a medical imaging data system including a motion rod, according to the teachings of the present disclosure;
Figure 6A is an illustration of medical imaging data related to a target anatomy including a motion rod and including a shape model with respect to a boundary of the medical imaging data, according to the teachings of the present disclosure;
Figure 6B is an illustration of medical imaging data related to a target anatomy including a shape model with respect to a boundary of the medical imaging data, according to the teachings of the present disclosure;
Figure 6C is an illustration of medical imaging data related to a three-dimensional model of a bone comprising a mesh surface, according to the teachings of the present disclosure;
Figure 7 is an illustration of the intensity histogram of a medical image, according to the teachings of the present disclosure;
Figure 8 is an illustration of the intensity distribution of fat and muscle for the identification of the fat intensity, according to the teachings of the present disclosure;
Figure 9 is an illustration of the bone mineral density calibration factor based on a relationship between the identified intensities and the bone mineral density equivalent values of the motion rod, air, and fat, according to the teachings of the present disclosure;
Figure 10 is a method flow chart illustrating example steps for the evaluation of a medical image exhibiting bone, air, fat, and a motion rod for the determination of the bone mineral density of the bone, according to the teachings of the present disclosure; and
Figure 11 is a method flow chart illustrating example steps of the identification of the intensity of fat in a medical image, according to the teachings of the present disclosure.

### DETAILED DESCRIPTION

Described herein are systems, computer-implemented methods, software programs, non-transitory computer readable media and/or techniques for automatically evaluating medical imaging data. Reference will now be made in detail to the various examples of the present disclosure illustrated in the accompanying drawings. Wherever possible, the same or like reference numbers will be used throughout the drawings to refer to the same or like features. It should be noted that the drawings are in simplified form and are not drawn to precise scale.

### Medical System

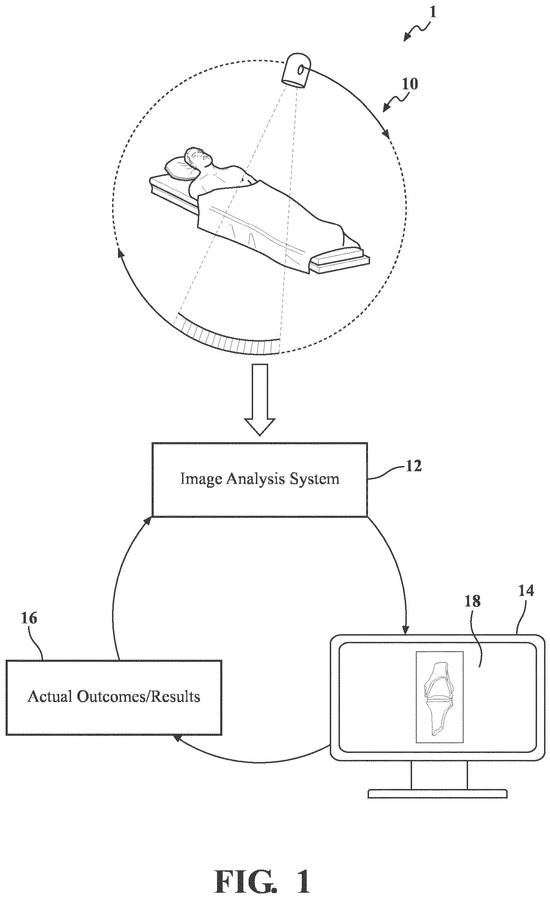

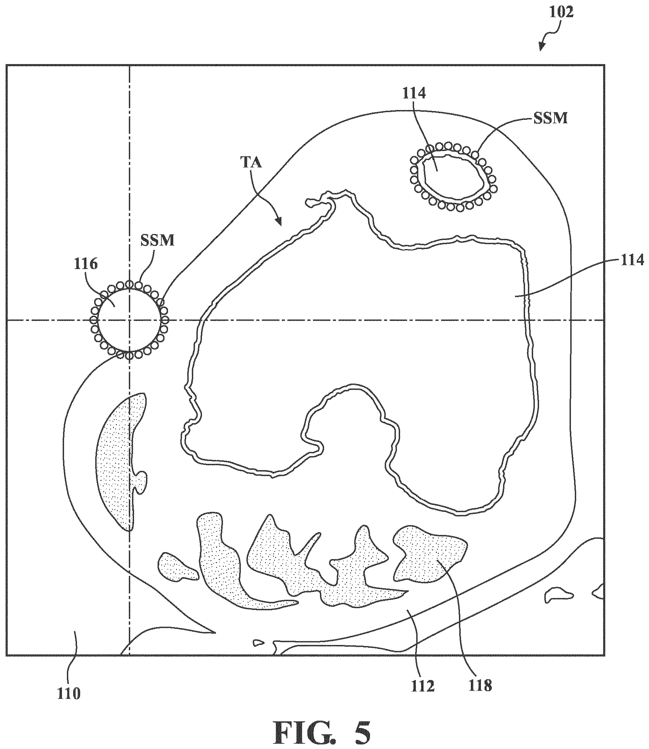

FIG. 1 illustrates a medical system 1 for evaluating a medical image 102 (Seen in FIG. 5) exhibiting air 110, fat 112, bone 114, and a motion rod 116. Specifically, the medical system 1 may evaluate a medical image 102 to determine a bone mineral density (BMD) of the bone 114 based on a phantomless bone mineral density calibration of the medical image 102. The medical system 1 may include a greater or fewer number of components than depicted.

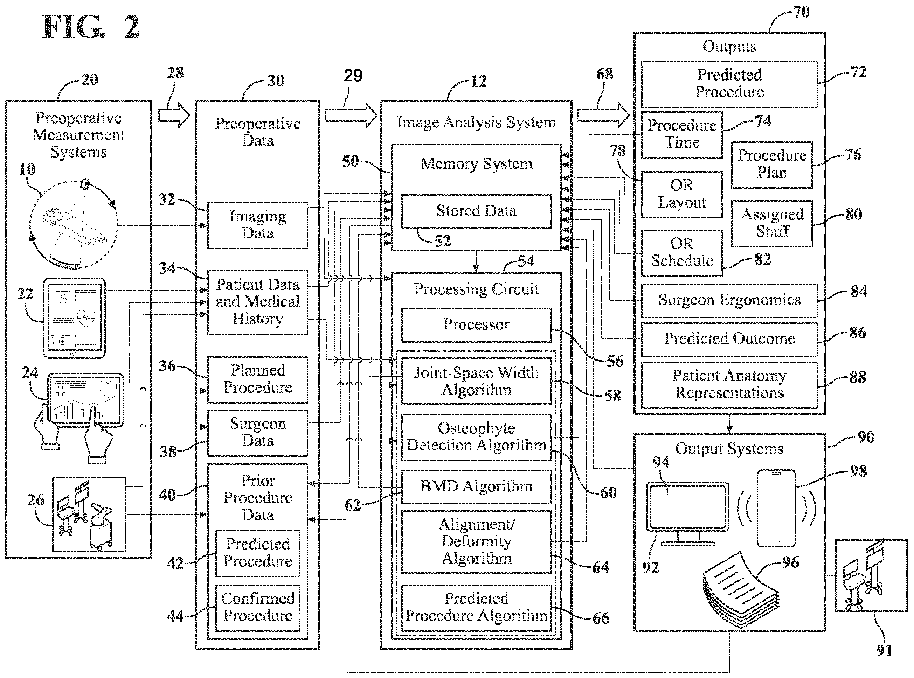

Referring to FIG. 1, the medical system 1 may include a diagnostic imaging device 10 (e.g., a computed tomography or CT scanner), an image analysis system 10, and an electronic display 21. A patient who is planning to undergo a procedure (e.g., surgery) may first undergo imaging using the diagnostic imaging device 10. The image analysis system 12 may analyze images and/or information collected during imaging (which may be transmitted from or stored in the device 10) to determine various outputs 70 (FIG. 2) and generate graphical user interfaces (GLTIs) 18 to display on a display 14. The image analysis system 12 may further determine recommended procedures (e.g., a predicted procedure), procedure logistics (e.g., procedure scheduling), and/or predicted outcomes (e.g., a risk of complication during the procedure or a risk of infection post-procedure) that are based on the determined outputs 70. As the course of treatment is continued, actual outcomes and/or results 16 may also be used by the image analysis system 12 to either update its predictions and/or to make future predictions for future patients. The image analysis system 12 may be implemented as one or more computer systems or cloud-based electronic processing systems. Details of the image analysis system 12 are discussed with reference to FIG. 2.

Referring to FIG. 2, the medical system 1 may include one or more preoperative measurement systems 20 which collect and/or output (via arrow 28) preoperative data 30 about the instant patient and/or prior patients (e.g., similar prior patients). The image analysis system 12 may receive (via arrow 29) and analyze the preoperative data 30 to generate one or more outputs or determinations 70, which may be output (via arrow 68) to one or more output systems 90. The medical system 1 can include non-transitory memory for storing instructions, which when executed by one or more processors, execute software, modules, or programs that can perform any of the aspects described herein. The processes herein can be implemented by any control system, one or more controllers, or any one or more processing devices.

Each of the components of the medical system 1 can operate as to send and/or receive data. Components that are communicatively coupled may include components capable of exchanging data signals with one another such as, for example, electrical signals via conductive medium, electromagnetic signals via air, optical signals via optical waveguides, and the like. Additionally, it is noted that the term "signal" means a waveform (e.g., electrical, optical, magnetic, mechanical, or electromagnetic), such as DC, AC, sinusoidal-wave, triangular-wave, square-wave, vibration, and the like, capable of traveling through a medium. As used herein, the term "communicatively coupled" means that coupled components are capable of exchanging signals with one another such as, for example, electrical signals via conductive medium, electromagnetic signals via air, optical signals via optical waveguides, and the like.

The preoperative measurement systems 20 may include the imaging device 10, electronic devices storing electronic medical records (EMR) 22; patient, practitioner, and/or user interfaces or applications 24 (such as on tablets, computers, or other mobile devices); and a robotic and/or automated data system or platform 26 (e.g., MAKO Robot System or platform, MakoSuite, etc.), which may include a robotic device 142. The medical system 1 may collect current imaging data 32 via the imaging device 10 and supplemental or additional information via the EMR 22, interfaces 24, sensors and/or electronic medical devices, and/or robotic platform 26.

The imaging device 10 may be configured to collect or acquire one or more images, videos, or scans of a patient's internal anatomy, such as bones, ligaments, soft tissues, brain tissue, etc., to generate imaging data 32, which will be described in more detail later. The imaging device 10 may include a computed tomography (CT) scanner (e.g., a supine CT scanner). The imaging device 10 may include, in addition to a CT scanner, a magnetic resonance imaging (MRI) machine, an x-ray machine, a radiography system, an ultrasound system, a thermography system, a tactile imaging system, an elastography, nuclear medicine functional imaging system, a positron emission tomography (PET) system, a single-photon emission computer tomography (SPECT) system, a camera, etc. The collected images, videos, or scans may be transmitted, automatically or manually, to the image analysis system 10. In some examples, a user may select specific images from a plurality of images taken with an imaging device 10 to be transmitted to the image analysis system 10.

The electronic data processing system 1 may use previously collected data from EMR 22, which may include patient data and medical history 34. The electronic data processing system 1 may also collect present or current (e.g., in real time) patient data via patient, practitioner, and/or user interfaces or applications 24. User interfaces 24 may also collect clinical data such as planned procedure 36 data and planned surgeon and/or staff data 38. These user interfaces 24 may be executed on and/or combined with other devices disclosed herein (e.g., with robotic platform 26). The electronic data processing system 1 may collect prior procedure data 40 from prior patients and/or other real-time data or observations (e.g., observed patient data 34) via robotic platform 26. The prior procedure data 40 may include prior predicted procedures 42 and prior confirmed procedures 44.

Although the preoperative measurement system(s) 20 is described in connection with imaging device 10, EMR 22, user interfaces 24, and robotic platform 26, other devices may be used preoperatively to collect preoperative data 30. For example, mobile devices such as cell phones and/or smart watches may include various sensors (e.g., gyroscopes, accelerometers, temperature sensors, optical or light sensors, magnetometer, compass, global positioning systems (GPS) etc.) to collect patient data 34. Other types of systems or devices that may be used in the preoperative measurement system 20 may include electromyography or EMG systems or devices, motion capture (mocap) systems, sensors using machine vision (MV) technology, virtual reality (VR) or augmented reality (AR) systems, etc.

The preoperative data 30 may be data collected, received, and/or stored prior to an initiation of a medical treatment plan or medical procedure. As shown by the arrows in FIG. 2, the preoperative data 30 may be collected using the preoperative measurement systems 100, from memory system 50 (e.g., cloud storage system) of the image analysis system 10, and from output systems 90 (e.g., from a prior procedure) for one or more continuous feedback loops.

As previously described, the preoperative data 30 may include imaging data 32, patient data and/or medical history 34, information on a planned procedure 36, surgeon data 38, and prior procedure data 40.

The imaging data 32 may include one or more images (e.g., raw images), videos, or scans of a patient's anatomy collected and/or acquired by the imaging device 10. The image analysis system 12 may receive and analyze one or more of these images to determine further imaging data 32, which may be used as further input preoperative data 30. In some examples, imaging device 10 may analyze and/or process the one or more images and send any analyzed and/or processed imaging data to the image analysis system 12 for further analysis.

The one or more images of the imaging data 32 may illustrate or indicate, and the image analysis system 12 may be configured to identify and/or recognize, in the images: bone, cartilage, or soft tissue positions or alignment, composition or density, fractures or tears, bone landmarks (e.g., condyle surface, head or epiphysis, neck or metaphysis, body or diaphysis, articular surface, epicondyle, lateral epicondyle, medial epicondyle, process, protuberance, tubercle vs tuberosity, tibial tubercle, trochanter, spine, linea or line, facet, crests and ridges, foramen and fissure, meatus, fossa and fovea, incisure and sulcus, and sinus), geometry (e.g., diameters, slopes, angles) and/or other anatomical geometry data such as deformities or flare (e.g., coronal plane deformity, sagittal plane deformity, lateral femoral metaphyseal flare, or medial femoral metaphyseal flare). Such geometry is not limited to overall geometry and may include relative dimensions (e.g., lengths or thicknesses of a tibia or femur).

The one or more images of the imaging data 32 may indicate bone quality and/or density or other measures of bone health. Bone density may be determined using the image analysis system 10, as described in further detail below.

Preoperative data 30 may include any other additional or supplemental information stored in memory system 20, which may also include known data and/or data from third parties, such as data from the Knee Society Clinical Rating System (KSS) or data from the Western Ontario and McMaster Universities Osteoarthritis Index (WOMAC).

The image analysis system 12 may be an artificial intelligence (Al) and/or machine learning system that is "trained" or that may learn and refine patterns between preoperative data 30, outputs 70, and actual results 16 (FIG. 1) to make determinations. The image analysis system 12 may be implemented using one or more computing platforms, such as platforms including one or more computer systems and/or electronic cloud processing systems.

The image analysis system 12 may further include the memory system 50 and a processing circuit 54. The memory system 50 may have one or more memories or storages configured to store or maintain the preoperative data 30, outputs 70, and stored data 52 from prior patients and/or prior procedures. The preoperative data 30 and outputs 70 of an instant procedure may also become stored data 52 Although certain information is described in this specification as being preoperative data 30 or outputs 70, due to continuous feedback loops of data (which may be anchored by memory system 20), the preoperative data 30 described herein may alternatively be determinations or outputs 70, and the determined outputs 70 described herein may also be used as inputs into the image analysis system 10. For example, some preoperative data 30 may be directly sensed or otherwise received, and other preoperative data 30 may be determined, processed, or output based on other preoperative data 30. Although the memory system 50 is illustrated close to processing circuit 54, memory system 50 may include memories or storages implemented on separate circuits, housings, devices, and/or computing platforms and in communication with image analysis system 10, such as cloud storage systems and other remote electronic storage systems.

The memory system 50 may include one or more external or internal devices (random access memory or RAM, read only memory or ROM, Flash-memory, hard disk storage or HDD, solid state devices or SSD, static storage such as a magnetic or optical disk, other types of non-transitory machine or computer readable media, etc.) configured to store data and/or computer readable code and/or instructions that completes, executes, or facilitates various processes or instructions described herein. The memory system 50 may include volatile memory or non-volatile memory (e.g., semiconductor-based memory device, a magnetic memory device and system, an optical memory device and system, fixed memory, or removable memory). The memory system 50 may include database components, object code components, script components, or any other type of information structure to support the various activities described herein. In some instances, the memory system 50 may be communicably connected to the processing circuit 54 and may include computer code to execute one or more processes described herein. The memory system 50 may contain a variety of modules, each capable of storing data and/or computer code related to specific types of functions.

The processing circuit 54 may include a processor 56 configured to execute or perform one or more algorithms based on received data, which may include the preoperative data 30 and/or any data in the memory system 50 to determine the outputs 70. The preoperative data 30 may be received via manual input, retrieved from the memory system 20, and/or received direction from the preoperative measurement systems 100. The processor 56 may be configured to determine patterns based on the received data.

The processor 56 may be implemented as a general purpose processor or computer, special purpose computer or processor, microprocessor, digital signal processor (DSPs), an application specific integrated circuit (ASIC), one or more field programmable gate arrays (FPGAs), a group of processing components, processor based on a multi-core processor architecture, or other suitable electronic processing components. The processor 56 may be configured to perform machine readable instructions, which may include one or more modules implemented as one or more functional logic, hardware logic, electronic circuitry, software modules, etc. In some cases, the processor 56 may be remote from one or more of the computing platforms comprising the image analysis system 10. The processor 56 may be configured to perform one or more functions associated with the image analysis system 10, such as precoding of antenna gain/phase parameters, encoding and decoding of individual bits forming a communication message, formatting of information, and overall control of one or more computing platforms comprising the image analysis system 10, including processes related to management of communication resources and/or communication modules.

In some aspects, the processing circuit 54 and/or memory system 50 may contain several modules related to medical procedures, such as an input module, an analysis module, and an output module. The image analysis system 12 need not be contained in a single housing. Rather, components of the image analysis system 12 may be located in various different locations or even in a remote location. Components of the image analysis system 10, including components of the processing circuit 54 and the memory system 20, may be located, for example, in components of different computers, robotic systems, devices, etc. used in surgical procedures.

The image analysis system 12 may use the one or more algorithms to make intermediate determinations and to determine the one or more outputs 70. The one or more algorithms may be configured to determine or glean data from the preoperative data 30, including the imaging data 32. For example, the one or more algorithms may be configured for bone recognition, soft tissue recognition, and/or to make determinations related to the intermediate imaging data 32 previously described. The one or more algorithms may operate simultaneously and/or separately to determine the one or more outputs 70 and/or display.

The one or more algorithms may be algorithms that are trained using, for example, linear regression, random forest regression, CatBoost regression, statistical shape modelling or SSM, etc. The one or more algorithms may be continuously modified and/or refined based on actual outcomes and/or results 16 (FIG. 1). The one or more algorithms may be configured to use segmentation techniques and/or thresholding techniques on received images, videos, and/or scans of the imaging data 32 to determine the previously described intermediate imaging data 32 and/or the one or more outputs 70. For example, the one or more algorithms may be configured to segment an image (e.g., a CT scan), threshold soft tissue, generate one or more plain text (e.g., .txt) comparisons of certain identified bones or tissues (e.g., tibia and femur), and run code to extract values (e.g., PPT or PTT) and populate a database. The one or more algorithms may be configured to automate data extraction and/or collection upon receiving an image from the imaging device 10.

The one or more algorithms may include a joint-space width algorithm 58, an osteophyte detection algorithm 60, an alignment/deformity algorithm 64, a predicted procedure algorithm 66, and a bone mineral density (BMD) algorithm 62. Alternatively, one or more of these algorithms may be combined. Each of the one or more algorithms may be configured to use image processing techniques to recognize or detect bones, tissues, bone landmarks, etc. and calculate or predict dimensions and/or positions thereof based on images acquired by the imaging device 10. The one or more algorithms may operate simultaneously (or alternatively, at different times throughout the preoperative and intraoperative periods) and exchange inputs and outputs.

The one or more outputs 70 may include bone mineral density of the imaging data 32. In implementations, the bone mineral density can be used to output a predicted procedure 42, a predicted procedure time or duration 74, a procedure plan 76, an operating room layout 78, an operating room schedule 82, assigned or designated staff 80, recommended surgeon ergonomics 84, predicted outcomes 86 of the procedure, and patient anatomy representations 88, which may include determined and/or enhanced images displayed on the display 14. The outputs 70 may be output electronically (e.g., on display 92, GUI 94, a mobile device 98, or any other monitors or displays which may be part of procedure systems 91) or printed physically (e.g., on paper, canvas, or film 96 or other materials via a printer). The medical system 1 can include any of the features or components of the system described in US Provisional Patent App No. 63/709,057, entitled "SYSTEMS, DEVICES, AND METHODS FOR PREDICTING TOTAL KNEE ARTHROPLASTY, PARTIAL KNEE ARTHROPLASTY, AND OTHER MEDICAL PROCEDURES", and filed October 18, 2024, which is hereby incorporated by reference.

### Generation of Medical Image and Bone Models including Bone Mineral Density

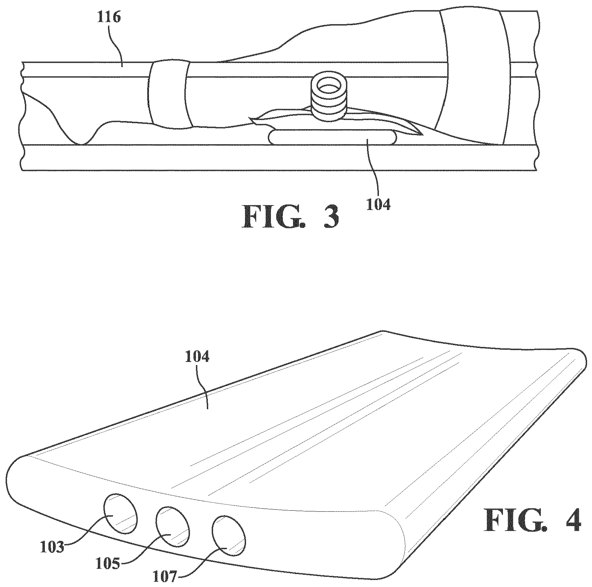

Referring now to FIGS. 3-9, the bone mineral density (BMD) algorithm 62 and/or the other one or more algorithms may analyze imaging data 32 to determine one or more outputs 70 to display within a GUI 25 such as a BMD of a patient's bone. BMD may refer to an amount of bone mineral present in the bone tissue of a bone and may be factored into a variety of medical treatments and/or used as an indicator of various medical conditions, such as osteoporosis or osteopenia. For example, BMD may be used for bone health assessments, risk predictions, monitoring treatment and disease, and preoperative planning for surgeries such as knee and hip replacements, among others.

BMD is often clinically measured by proxy through an analysis of medical images. However, a BMD typically may not be derived from a medical image directly and may require additional processing and/or calibration. For example, in many instances, a BMD of a bone 114 may be determined using a medical image 102 generated by a computed tomography (CT) scan including the bone 114 and a calibration device, such as a calibration phantom 104. A calibration phantom 104 may include a plurality of different reference materials (e.g., reference materials 103, 105, 107) for each of which a BMD equivalent is known, and may be placed near the bone 114 during the CT scan. The medical image 4102 generated by the CT scan represents the attenuation of x-rays as they pass through different materials, indicated by different intensities exhibited in the medical image 4102 (with higher intensities appearing lighter and lower intensities appearing darker, in this example). At least one reference material of the plurality of different reference materials included in the calibration phantom 104 is configured to cause a different attenuation of the x-rays than the bone 114, thereby producing a different intensity exhibited in the medical image 4102 than that of the bone, and because the BMD equivalent of the at least one reference material is known, the intensity of the at least one reference material and the BMD equivalent of the at least one reference material may be used as reference points to determine the BMD of the bone 114. However, such calibration phantoms 104 are often unavailable and/or uncomfortable for the patient. Thus, a method for determining a BMD of a bone from a medical image that is more accessible, reliable, accurate, and comfortable than using a calibration phantom is desired.

Referring to FIGS. 3-9, BMD algorithm 62 and/or the one or more algorithms may be configured to determine a BMD of a patient's bone from an acquired image 102 using a BMD equivalent from only a single reference material included in a calibration device. For example, the calibration device may be a motion rod 116. A motion rod 116 may be a straight rod or bar that is physically coupled to a patient's anatomy or limb (e.g., the patient's leg) during a medical imaging process (e.g., a CT scan or an MRI scan) to hold the patient's anatomy still or straight. Additionally, or alternatively, to ensure the accuracy of a medical imaging process, a motion rod 116 may be used to determine if a patient's anatomy remained still or straight during the medical imaging process. For example, any motion of the motion rod 116 detected during a medical imaging process may indicate that a patient's anatomy was moved during the medical imaging process, which may require a rejection or repeat of the medical imaging process. A motion rod 116 may be included as a standard component of a medical imaging process protocol. A motion rod 116 may be radiopaque and may made of aluminum or any other suitable material.

In one example, the BMD algorithm 62 may be configured to determine a BMD of a patient's bone from an acquired image 102 using a BMD equivalent from only a single reference material included in a calibration device (e.g., a motion rod 116) by first identifying, from within the acquired image 102, intensities of the single reference material included in the calibration device, a bone 114 of the patient's anatomy, and at least one of fat 112 from the patient's anatomy and air 110. In this example, the BMD algorithm 62 then receives, accesses, retrieves, or otherwise obtains predetermined BMD equivalents for the single reference material included in the calibration device and the at least one of the fat 112 and the air 110. Then, using the intensities and the BMD equivalents of the single reference material included in the calibration device and the at least one of the fat 112 and the air 110, the BMD algorithm 62 determines a BMD calibration factor (BCF) for the acquired image 102. The BCF may be determined using a linear regression, a best fit line, or any other suitable method. Since both (i) the single reference material included in the calibration device and (ii) the at least one of the fat 112 and the air 110 may typically be present in a medical image, using the intensities and BMD equivalents of the single reference material included in the calibration device and the at least one of the fat 112 and the air 110 to determine the BCF may be more reliable than using a calibration phantom 104 (as described above). Furthermore, the single reference material included in the calibration device and the at least one of the fat 112 and the air 110 may produce a wider range of intensities than a calibration phantom 104, thereby producing a more accurate BCF. After determining the BCF, the BMD algorithm 62 may apply the BCF to the intensity (i.e. the CT image data) of the bone 114 to determine the BMD of the bone 114. The BMD of the bone 114 may then be used to generate one or more GUIs. For example, the BMD of the bone 114 may be used to generate and display an artificial or virtual (e.g., two-dimensional or three-dimensional) model of the patient's anatomy, exhibiting the BMD of the bone 114, within a GLTI.

In some instances, in which intensities of only one of fat 112 and air 110 is used to generate the BCF, the BMD algorithm 62 may be configured to compare the intensity and/or the BMD equivalent of the one of the fat 112 and the air 110 that was not used in determining the BCF to one or more relationships between the intensities and the BMD equivalents of the single reference material included in the calibration device and the one of the fat 112 and the air 110 that was used in determining the BCF, to determine if the BCF is acceptably accurate. For example, if the intensity and/or the BMD equivalent of the one of the fat 112 and the air 110 that was not used in determining the BCF does not fit, within an acceptable threshold, a linear regression or a best fit line generated using the intensities and the BMD equivalents of the single reference material included in the calibration device and the other of the fat 112 and the air 110, the BMD algorithm 62 may determine that the BCF is not acceptably accurate. In some instances, intensities and BMD equivalents of the single reference material included in the calibration device, the fat 112, and the air 110 are used to determine the BCF. In some instances, the bone 114, the single reference material included in the calibration device, and the fat 112 and/or the air 110, and/or their respective intensities, may be identified from within the acquired image 102 using a shape model.

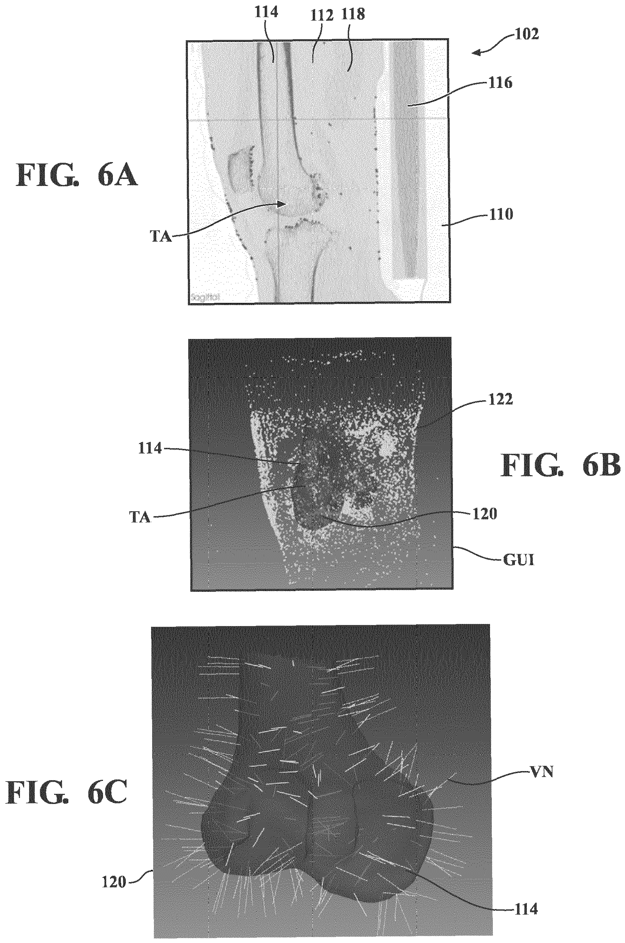

Referring to FIGS. 6A-6B, the image analysis system12automatically identifies and fits a shape model SSM to target anatomy TA in the medical imaging data 32. The term "shape model" is used herein to include any one or more of: a statistical shape model, an active shape model, an active appearance model, an active contour model, or any other suitable type of shape model. In one example, the shape model SSM can be understood as a mesh, shape or contour that has adjustable nodes to deform the mesh, shape, or contour to substantially confirm to the shape or contour of the target anatomy TA in the medical imaging data 32. The shape model can be initially derived or generated from a population of other patient images exhibiting similar anatomies as the target anatomy TA. The population can exhibit similar characteristics of the subject patient, such as age, gender, ethnicity, size, or other physical or demographical data. For instance, when the target anatomy TA is a bone, the shape model SSM may be derived from a statistical representation of images of bones of comparable anatomical origin from a group of patients known to have normal or "healthy" bone anatomy. The shape data used to derive the shape model SSM may include geometric characteristics of a bone such as landmarks, surfaces, boundaries, geometric characteristics, or intensity information of a target anatomy TA. The shape data can be provided from analysis of other patient images and/or from point clouds acquired from normal bones of comparable anatomical origin. The image analysis system12can select one shape model from among a plurality of shape models that is best fit to the target anatomy. The best-fit shape model may or may not need to be morphed to the target anatomy. Alternatively, image analysis system12can utilize one generic shape model (for the specific anatomy type) and morph it to the target anatomy. In any implementation described herein, the shape model may be a singular shape model or may be realized as a plurality of shape model instances.

The shape model SSM can be utilized by using an algorithm that automatically segments the medical imaging data 32. In one implementation, the SSM may represent "healthy" anatomy that may not necessarily correspond exactly to the target anatomy of the subject patient. The segmentation algorithm may perform image processing such as alignment of coordinates of the target anatomy TA in image data to the SSM. The alignment may be based on key marker points on the target anatomy TA in image data and the SSM. The segmentation algorithm may then morph, deform, or scale the SSM until the SSM and the target anatomy TA in the image data register. This fitting process can be performed using any optimization technique, such as a least squares optimizer. The registration may include adjusting the size and shape of the SSM to the target anatomy TA in image data and adjust a location of the SSM to align with the target anatomy TA in image data. The result of the registration may be a shape model that approximates the target anatomy TA in image data (e.g., optionally with or without osteophytes or abnormal morphology).

In another implementation, the automated segmentation algorithm performs an initial segmentation process on the image data associated with the target anatomy TA with a first shape model to generate an initial segmentation of the target anatomy TA. The automated segmentation can optionally further perform a refined segmentation process on the image region of the image data associated with the target anatomy TA using a neural network that takes as an input the image data of the target anatomy TA and the output of the initial segmentation. The first shape model can be mapped to an output of the refined segmentation process. Optionally, anatomical landmarks AL of the target anatomy TA can be identified. Examples of such automated segmentation may be implemented in a manner as described in U.S. Provisional Patent App. No., 63/505,466, filed June 1, 2023, and entitled "Segmentation of Bony Structures" (Attorney Docket No. 060939.01029), the entire contents of which are hereby incorporated by reference.

As shown in FIG. 5, the shape model SSM (derived from any techniques described above) is represented as a shape that approximates the outline of the target anatomy TA (e.g., knee joint bone(s)) relative to the medical imaging data 32. The process of visualizing the shape model SSM relative to the image is optional and not necessary for this automated pre-check. If useful, a technician may manually retrieve and review any shape model relative to any corresponding image on the GLTI.

Additionally, the image analysis system12can create a shape model (e.g., a cylinder or circle) to fit to the motion rod 116 or it's cross-section (if slices are used). For volumetric analysis, the image analysis system12can check whether the full cylinder is present in the 3D volume and matches the scanned motion rod parameters. The full cylinder indicates that the motion rod 116 was stationary and hence the volume was taken while the patient was motionless. If a geometry other than a full cylinder (e.g., such as a partial cylinder, or a blurred geometry) is identified in the volume, the image analysis system 12automatically identifies that the motion rod 116 had moved during scanning and hence the medical image 102 was taken while the patient was moving. If slices are used, the image analysis system12can check whether a full circle is present in the slice at the location of the motion rod 116. The full circle indicates that the motion rod 116 was stationary and hence the slice was taken while the patient was motionless. If a geometry other than a full circle (e.g., such as a partial circle, or a blurred geometry) is identified in a particular slice, the image analysis system12automatically identifies that the motion rod 116 had moved during scanning and hence the slice was taken while the patient was moving.

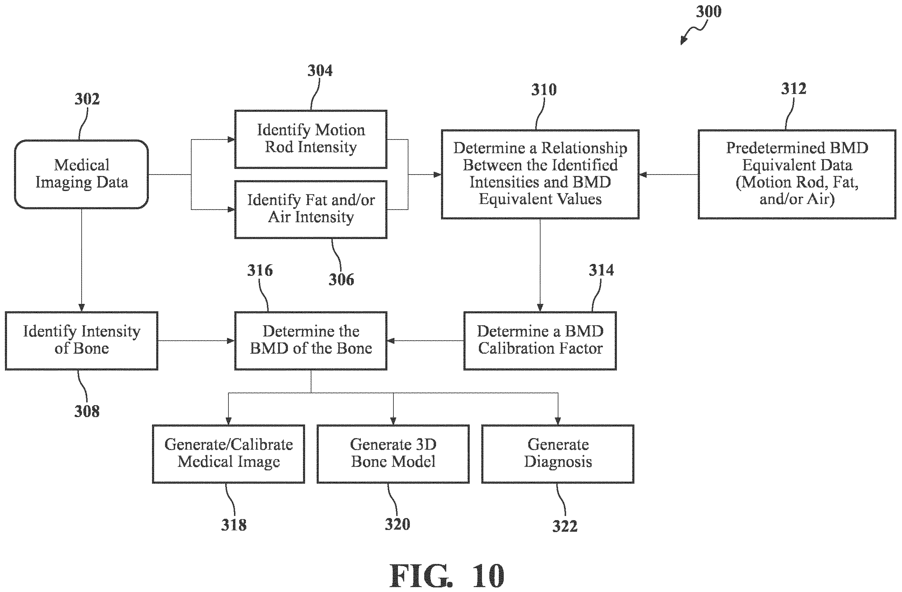

Referring now to FIG. 10, a flowchart 300 illustrates an example method for evaluating medical image data 32 that includes air 110, fat 112, bone 114, and the motion rod 116 to determine the BMD of the bone based on a phantomless BMD calibration. As described above, the method may be carried out by the image analysis system 12 of the medical system 1. The flowchart 300 depicted in FIG. 10 is a representation of a machine readable instruction set stored in memory and executed by processors 56 of the medical system 1. The processes depicted of the flowchart 300 may be executed at various times and in response to inputs or other signals from the GUI or other user initiated signal. In other implementations, the process of the flowchart 300 may be executed as directed by a remote source or otherwise automatically without the input of a user.

At step 302 the logic executed by the processor 56 of the image analysis system 12 causes the medical system 1 to generate or receive the medical image 4102, as described above. As an example, a patient may be positioned within the medical imaging device 10 with knees at full extension. The medical imaging device 10 generates the initial medical image data 32, for example, a CT scan. In implementations, an antero-posterior and later views are used to check alignment and CT scans may be performed at the hip, the knee, and the ankle joint locations, as seen in FIG. 4. Knee joint acquisition includes coverage superior to the patella to the tibial tuberosity with contiguous 0.5-1mm slices. In implementations, scanning parameters use a bone reconstruction with tube cottage of 120-140 kVp and tube current of 200-400 mA with auto-exposure control. Coverage by the scan may include 10cm on each side of the knee j oint line. The elements of the CT medical image can be identified using the shape model SSM described above and outputting in a gray scale image.

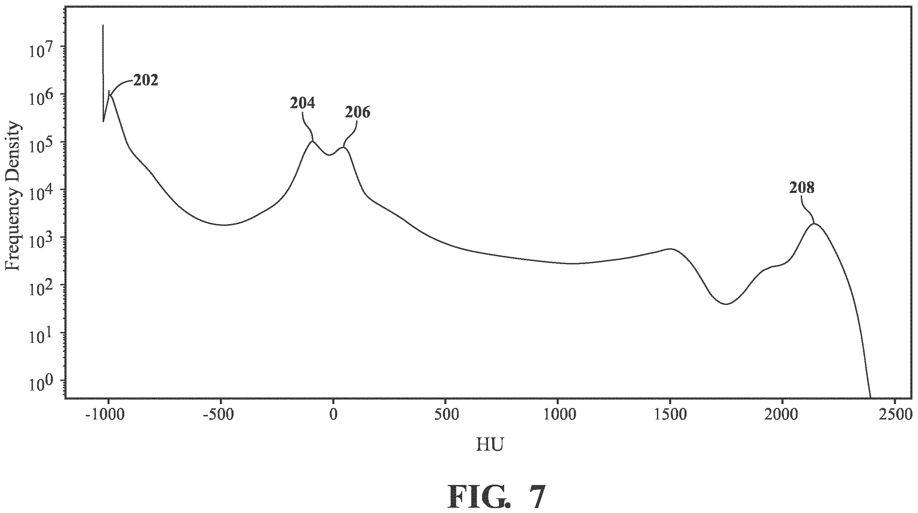

At steps 304, 306, and 308, the image analysis system 12 identifies, from the medical image data 32, intensities of the bone 114, the motion rod 116, and at least one of the air 110 and/or the fat 112. As seen in FIG. 7, the intensity distribution of the air 110, the tissues including the fat 112, the bone 114, and muscle 118, and the motion rod 116 is illustrated in a histogram analysis of the images. Using the peaks of the intensity distribution, including an air peak 202, a fat peak 204, a muscle peak 206 and a motion rod peak 208, the intensity of each of the above features can be determined from the medical image data 32. The processes for identifying intensities from the identification the air peak 202, the fat peak 204, and the motion rod peak 208 are described in more detail below.

Still at step 306, to determine the intensity of air 110, the image analysis system 12 finds the air peak 202 on the intensity distribution of FIG. 7. The image analysis system 12 identifies image voxels containing air using histogram analysis of the medical image 102, as seen in FIG. 6. Both air voxels, and voxels outside reconstructed CT field appear black in the medical image, however, the outside voxels are generally coded as the lowest HU values. To identify the HU value of the air 110, the first single largest peak of the histogram is eliminated. In implementations, small narrow peaks associated with noise are also removed from the histogram. The remaining peak with the lowest HU value is identified as the air peak 202 to be used for the intensity of the air.

To identify the intensity of the motion rod 116, an active appearance model (AAM) may be used to enable accurate cartilage and bone segmentations. The AAM may be trained using random samples of images from a dataset. The non-varying shape of the motion rod 116 means that small training sets may be used. The motion rod 116 in each training image was manually segmented using intensity thresholding to produce surfaces for AAM training. Rod surfaces resulting from image search by AAM were eroded to exclude partial voxels and beam hardening. The median voxel intensity from the segmented rod volume may be used as the rod HU value for the calibration standard for the motion rod 116 to obtain the intensity. A method for identifying the intensity of the fat 112 with be described in greater detail below.

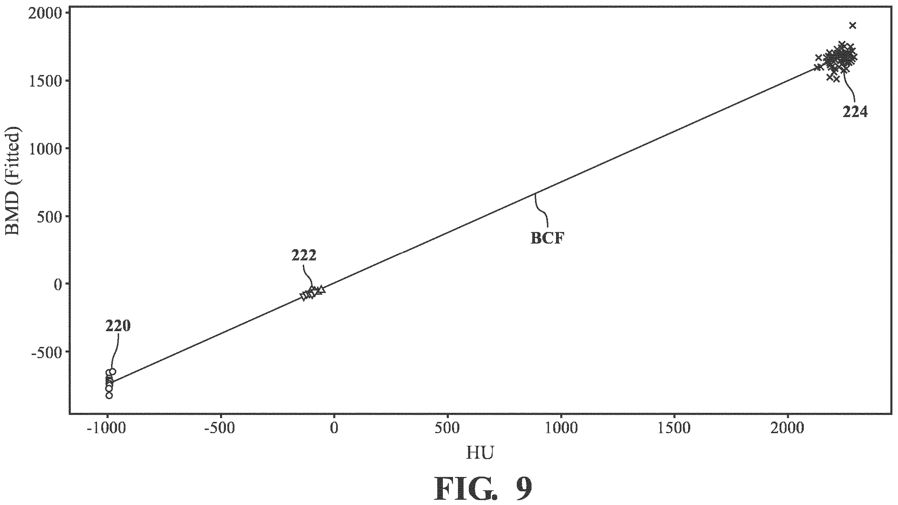

At step 312, the medical image analysis system 12 receives predetermined BMD equivalent data for the motion rod 116, and at least one of the air 110 and/or the fat 112. The predetermined BMD equivalent data is density data that is equivalent to the BMD. This data is generally stable for each of the motion rod 116, the air 110, and the fat 112. In implementations, this data may be obtained using a BMD calibration phantom 104. The predetermined BMD equivalent data may be plotted against the identified intensity for the motion rod 116 and the at least one of the air 110 and/or the fat 112. Such a plot is shown in FIG. 9 displaying the values for air 110, fat 112, the motion rod 116 in addition to the values of the calibration phantom 104 reference materials 103, 105, 107.

At steps 310 and 322, the image analysis system 12 determines a BMD calibration factor BCF based on a relationship between the identified intensities of the motion rod 116, and at least one of the air 110 and the fat 112, and the BMD equivalent values of the motion rod 116 and the at least one of the air 110 or the fat 112, as displayed in FIG. 9. The air intensity found by the air peak 202 and the BMD equivalent value may be plotted at point 220. The fat intensity found by the fat peak 204 and the BMD equivalent value is plotted at point 222. The motion rod intensity found by the motion rod peak 208 and the BMD equivalent value is plotted at point 224. In implementations, the BMD calibration factor BCF is found using a linear regression or a best fit line. In implementations, the BMD calibration factor BFC is the slope of the line. As described above, the BMD calibration factor BCF is determined without the use of a BMD calibration phantom. Instead, the BMD calibration factor BCF is determined using elements internal and external to the patient, i.e. the motion rod 116 the fat 112 and/or the air 110, of which are present in each scan of the patient to determine the BMD calibration factor. Further, the above elements have a wider range of HU units, therefore generating a more accurate linear relationship compared to using the BMD calibration phantom 104.

Knowing the BMD calibration factor, the image analysis system 12 can determine the BMD of the bone 114 identified with the shape model SSM at step 316. After the generation of the bone 114 BMD, at steps 318 and 320, the image analysis system 12 may generate a 3D bone model visualizing the BMD. In some implementations, the image analysis system 12 may either generate or calibrate the medical image 102 to reflect the new calibration. In other words, the medical image 102 is reconfigured to display a more accurate grey scale image displaying the BMD. In some implementations, at step 322, the medical system 1 may generate a diagnosis based on the determined BMD.

In implementations where only one of the fat 112 or the air 110 is used to generate the BMD calibration factor BCF, the image analysis system 12 may compare the intensity and the BMD equivalent value of the one of the air 110 or the fat 112 not used in determining the BMD calibration factor BCF to the relationship between the identified intensities and the BMD equivalent values of the motion rod 116 and the one of the air 110 or the fat 112. The intensity and the BMD equivalent value of the one of the air 110 or the fat 112 not used in determining the BMD calibration factor BCF generate a point on the graph of FIG. 9 displaying the BMD calibration factor BCF. The point of the one of air 110 or fat 112 not used is at a distance away from the BMD calibration factor BCF. The image analysis system 12 rejects the BMD calibration factor BCF as unacceptable if the comparison exceeds a threshold. This comparison is useful in determining if the medical image data 32 is reliable and improves the accuracy of the phantomless BMD calibration factor BCF.

In other implementations, each of the motion rod 116, the fat 112, and the air 110 is used to determine a BMD calibration factor. In such implementations, the intensities of the bone 114, the motion rod 116, the air 110, and the fat 112 are identified from the medical image data 32. As described above, the BMD equivalent value of the motion rod 116, the air, and the fat is obtained as a predetermined value. The BMD calibration factor BCF is determined based on a relationship between the identified intensities and the BMD equivalent values of the motion rod 116, the air 110, and the fat 112.

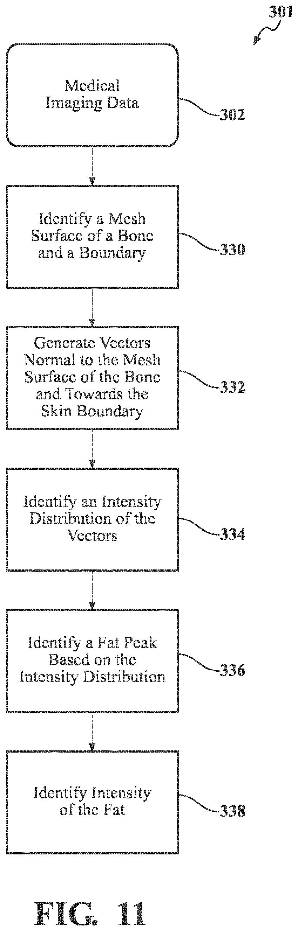

Referring now to FIG. 11, a flowchart 301 of an example method for identifying the intensity of fat in the medical image 102 exhibiting air 110, fat 112, the bone 114, and the motion rod 116 is depicted. As described above, the method may be carried out by the processor 56 of the image analysis system 10. The flowchart 301 depicted in FIG. 6 is a representation of a machine-readable instruction set stored in memory and executed by processors 56 of an image analysis system 10. The process of the flowchart 301 may be executed at various times and in response to inputs or other signals from the GUI or another user-initiated signal. In other implementations, the process of the flowchart 301 may be executed as directed by a remote source or otherwise automatically without the input of a user.

At step 302, as above the logic executed by the image analysis system 12 causes the image analysis system 12 to generate or receive medical image data 32, as described above. The elements of the CT medical image can be identified using the shape model SSM described above and outputting a three-dimensional (3D) model of the bone 114 comprising a mesh surface 120 as seen in FIGS. 6B-6C. At step 302, the image analysis system 12 identifies the mesh surface 120 of the bone 114 and a skin boundary 122 of the model using the shape model SSM. The image analysis system 12 then generates vectors normal VN to the mesh surface 120 of the bone 114 moving in a direction towards the skin boundary 122 at step 332.

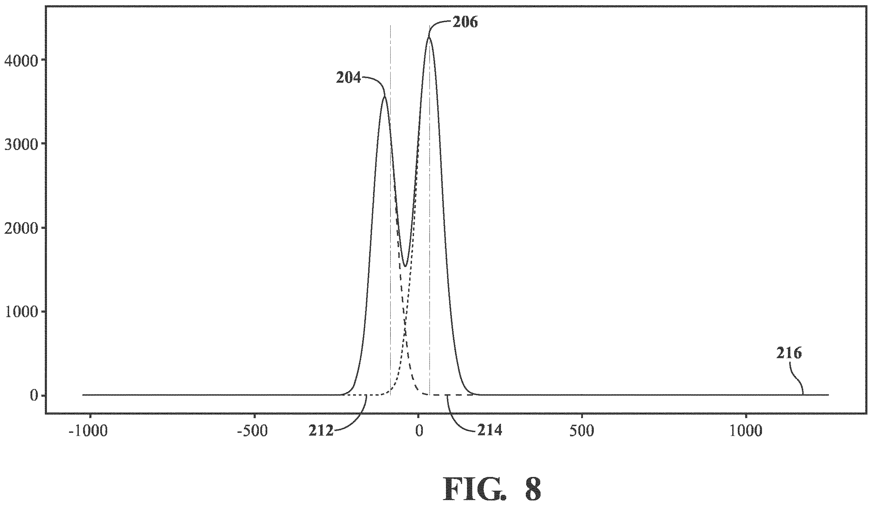

At step 334, the intensity distribution along each vector normal VN from the mesh surface 120 to the skin boundary 122 is identified and plotted, as seen in FIG. 8. In implementations, normal rays to the AAM segmented bone surface are projected from the centroid of each surface triable and sampled until a discontinuity in image intensity values is detected. The discontinuity represents a soft tissue boundary to air, bone, or cartilage. In implementation, an HU discontinuity of 300 HU along the normal vector is used to detect boundaries. Using the plot generated from the intensity distribution of the vectors normal VN, a combined histogram distribution 216 may be displayed.

At step 336, the fat peak 204 may be identified based on the histogram distribution of the intensity of the medical image 102. The combined histogram distribution 216 is the combined distribution of a fat component 214 and a muscle component 212. As shown, various peaks 204, 206 may be identified from the distribution. These peaks display the fat intensity at the fat peak 204 and a muscle intensity at the muscle peak 206 in addition to other tissues. The fat intensity peak 204 may be positioned to the left of the muscle intensity peak 206. After identifying the fat peak 204, at step 306, as above, the intensity of the fat 112 is obtained at step 338.

The identified intensities of the bone 114, as identified with the SSM described above, may be plotted on the linear regression fit of the BMD calibration factor BCF generated from the relationship between the identified intensities and a BMD equivalent values of the motion rod 116 and the at least one of the air 110 or the fat 112. From these obtained bone mineral densities, the gray scale medical image 102 can be recalibrated or a new medical image may be generated. Further a 3D bone model can be generated featuring the bone mineral densities. In implementations, upon determining the BCF, the bone mineral densities of the bone may be automatically determined and featured to the user, such as in the GLTI. Such data may be used in the execution of a surgical plan. For example, the location in which to screw, saw or drill in a bone. Further the data may be used to determine the speed in which a tool is set to cut through the bone. As another example, the bone mineral density can be used to automatically generate a diagnosis such as osteoporosis or a portion for clinical monitoring. A diagnosis may be based on a comparison of the values measures in the subject to normative reference values. In some implementations, the medical system 1 may include a navigated surgical system such as a robotic system that supports a tool that may manipulate a bone. The navigated surgical system may manipulate the bone based on the determined bone mineral density.

As evident from the forgoing description and figures, the phantomless BMD calibration by using the air, the fat, and the motion rod provided in the creation of each medical image for the execution of a surgical plan provide significant advantages and technical solutions by automatically, accessibly, accurately, and reliably allowing BMD calibration. The phantomless BMD calibration allows a user to obtain the BMD of the bone directly from the CT scan of the patient allowing for faster, easier results. The use of such phantomless elements provide a more accurate calibration factor as the range of the HU or intensities used is greater. Further, the elements are present in each medical image taken, therefore eliminating the need to have a phantom added, which often are lost and are uncomfortable for the patient. Furthermore, the motion rod, the fat, and the air each have stable BMD equivalent values leading to reliable BMD calibration and calculation.

Diagnosis of low BMD depends on the comparison of values measured in a subject to normative reference values. At the spine, these have been established for the use of QCT in diagnosis of osteoporosis and low bone mass. However, unlike the wrist, hip or spine, the knee joint is not usually susceptible to osteoporotic fracture and normative values for BMD have not been established for diagnostic thresholds. Using this method, a threshold could be calculated for various depths from the articulating surface in the femur or tibia using the normative data. Other advantages not specifically described herein are readily understood by those skilled in the art.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A non-transitory computer readable medium (50) comprising instructions configured to automatically determine bone mineral density (BMD) from a medical image (102) exhibiting air (110), fat (112), bone (114) and a motion rod (116), wherein the instruction, when executed by one or more processors (56), are configured to:
automatically identify (304, 306, 308), from the medical image (102), intensities of the bone (114), the motion rod (116) and at least one of: air (110) or fat (112);
automatically obtain (312) a BMD equivalent value of the motion rod (116) and the at least one of the air (110) or the fat (112);
automatically determine (314) a BMD calibration factor (BCF) based on a relationship between the identified intensities and the BMD equivalent values of the motion rod (116) and the at least one of the air (110) or the fat (112); and
automatically determine (316) the BMD of the bone (114) within the medical image (102) based on the BMD calibration factor (BCF).

2. The non-transitory computer readable medium (50) of claim 1, wherein the instructions, when executed by the one or more processors (56), are further configured to:
automatically identify (304, 306, 308), from the medical image, intensities of the bone (114), the motion rod (116), the air (110), and the fat (112);
automatically obtain (312) a bone mineral density equivalent value of the motion rod (116), the air (110), and the fat (112);
automatically determine (314) a BMD calibration factor (BCF) based on a relationship between the identified intensities and a BMD equivalent values of the motion rod (116), the air (110), and the fat (112); and
automatically determine (316) the BMD of the bone (114) within the medical image (102) based on the BMD calibration factor (BCF).

3. The non-transitory computer readable medium (50) of claim 1, wherein the instructions, when executed by the one or more processors (56), are further configured to:
automatically generate (320), from the medical image (102), a 3D model of the bone (114) comprising a mesh surface (120).

4. The non-transitory computer readable medium (50) of claim 3, wherein the instructions, when executed by the one or more processors (56), are further configured to:
automatically identify (330), from the medical image a skin boundary (122);
automatically generate (332) vectors normal (VN) to the mesh surface (120) of the bone (114) and towards the skin boundary (122);
automatically identify (334) an intensity distribution of the vectors (VN);
automatically identify a fat peak (304) based on the intensity distribution; and
automatically identify the intensity of the fat (112) based on the fat peak (304).

5. The non-transitory computer readable medium (50) of claim 1, wherein the BMD equivalent value of the motion rod (116), the air (110), and the fat (112) are predetermined.

6. The non-transitory computer readable medium (50) of claim 1, wherein the relationship is a linear relationship.

7. The non-transitory computer readable medium (50) of claim 1, wherein the instructions, when executed by the one or more processors (56), are further configured to:
automatically generate (320) a three-dimensional bone model exhibiting the determined BMD of the bone.

8. The non-transitory computer readable medium (50) of claim 1, wherein the instructions, when executed by the one or more processors (56), are further configured to:
automatically generate (318) a new medical image based on the BMD calibration factor (BCF); or
automatically calibrate (318) the medical image based om the BMD calibration factor.

9. The non-transitory computer readable medium (50) of claim 1, wherein the instructions, when executed by the one or more processors (56), are further configured to:
automatically compare a relationship between the intensity and the BMD equivalent value of the one of the air (110) or the fat (112) not used in determining the BMD calibration factor (BCF) to the relationship between the identified intensities and the BMD equivalent values of the motion rod (116), and the at least one of the air (110) or the fat (112); and
automatically reject the BMD calibration factor (BCF) as being unacceptable in response to a determination that the comparison exceeds a threshold.

10. The non-transitory computer readable medium (50) of claim 1, wherein the medical image (102) is taken from a computed tomography (CT) scan (10).

11. The non-transitory computer readable medium (50) of claim 1, wherein the motion rod (116) is a physical bar coupled to a patient's anatomy (TA), and optionally, wherein the motion rod (116) is comprised of aluminum.

12. The non-transitory computer readable medium (50) of claim 1, wherein the instructions, when executed by the one or more processors (56), are further configured to:
automatically determine the motion rod in the medical image (102) using a statistical shape model (SSM) or an active appearance model.

13. The non-transitory computer readable medium (50) of claim 1, wherein the instructions, when executed by the one or more processors (56), are further configured to:
automatically determine the bone (114) in the medical image (102) using a statistical shape model (SSM) or an active appearance model.

14. The non-transitory computer readable medium (20) of claim 1, further comprising:
automatically generate (322) a diagnosis for the identified bone based on the BMD determined by the BMD calibration factor (BCF).

15. A computer-implemented method for evaluating a medical image (102) exhibiting air (110), fat (112), bone (114) and a motion rod (116), wherein computer-implemented method comprises automatically performing the following:
identifying (304, 306, 308), from the image (102), intensities of the bone (114), the motion rod (116) and at least one of: air (110) or fat (112);
obtaining (312) a bone mineral density equivalent value of the motion rod (116) and the at least one of the air (110) or the fat (112);
determining (314) a BMD calibration factor (BCF) based on a relationship between the identified intensities and a BMD equivalent values of the motion rod (116) and the at least one of the air (110) or the fat (112); and
determining (316) the BMD of the bone (114) within the medical image (102) based on the BMD calibration factor (BCF).
